# EUROPÄISCHE PATENTSCHRIFT

(11) **EP 2 698 130 B1**
(45) Veröffentlichungstag und Bekanntmachung des Hinweises auf die Patenterteilung: **23.11.2016**
(21) Anmeldenummer: 13003967.0
(22) Anmeldetag: 08.08.2013
(51) Int. Cl.: A61F 2/90, A61F 2/06, A61F 2/07, A61F 2/82, D04C 1/06

(54) **Verfahren zum Herstellen eines Körperimplantats**
Method for producing a body implant
Procédé de fabrication d'un implant corporel

(30) Priorität: 16.08.2012 DE 102012016301
(43) Veröffentlichungstag der Anmeldung: 19.02.2014
(73) Patentinhaber: Admedes Schuessler GmbH, 75179 Pforzheim (DE)
(72) Erfinder: Scherrible, Frank, 75179 Pforzheim (DE); Budillon, Florent, 75179 Pforzheim (DE)
(74) Vertreter: Müller-Boré & Partner Patentanwälte PartG mbB

(56) Entgegenhaltungen:
- WO-A2-00/44308
- WO-A2-2008/112242

## Beschreibung

Die vorliegende Erfindung betrifft ein Verfahren zum Herstellen eines Körperimplantats, wie beispielsweise eines Stents, einer Gefäßstütze, eines Stentgrafts, eines Herzklappenrahmens, eines Okkluders, eines Flow Diverters oder dergleichen.

Derartige Körperimplantate werden beispielsweise durch Erzeugen von Textilstrukturen aus einem Material hergestellt. Dabei ist es schwierig, das Material der Textilstruktur mit anderen Materialien zu kombinieren, was wünschenswert wäre, beispielsweise zum Einbringen von Markern in Form von Garn oder Draht zum Erhöhen der Röntgensichtbarkeit oder dergleichen. Darüber hinaus sollte zumindest eines der Materialien mit Formgedächtniseigenschaften versehen werden, beispielsweise durch einen sogenannten Shape Setting Schritt.

DE 696 24 834 T2 offenbart ein Verfahren zur Herstellung einer Gefäßprothese bestehend aus metallischen und nicht-metallischen Garnen.

Gemäß dem Stand der Technik war es nicht möglich, bei einer Kombination von metallischen und nicht-metallischen Garnen eine Wärmebehandlung zum sogenannten Shape Setting durchzuführen. Daher war im Stand der Technik die Formgebung auf eine Formgebung durch plastische Verformung angewiesen. In diesem Zusammenhang wird auf die Druckschriften DE 696 24 834 T2 und DE 603 13 736 T2 verwiesen.

Jedoch ist eine Formgebung basierend auf einer Wärmebehandlung einer Formgebung basierend auf einer plastischen Verformung vorzuziehen, da bei einer plastischen Vorformung die Gefahr der Materialbeschädigung besteht. Es wurde erkannt, dass Bauteile, bei denen das Shape Setting idealer Weise durch eine Wärmebehandlung von zumindest metallischen Garnen erfolgt, andere mechanische Eigenschaften aufweisen, als dies bei plastisch verformten Garnen der Fall ist.

Bei Körperimplantaten kann beispielsweise das Fatigue-Verhalten durch plastisch verformte Garne negativ beeinflusst sein.

WO 00/44308A2 offenbart gewebte intravaskuläre Vorrrichtungen und Verfahren für deren Herstellung sowie ein Gerät für deren Einführvorgang.

WO 2008/112242 A2 offenbart ein gewobenes Erzeugnis mit Formgedächtniselementfäden.

Es ist daher Aufgabe der vorliegenden Erfindung eine Verbindung von mindestens zwei unterschiedlichen Materialien bzw. Materialeigenschaften innerhalb einer Textilstruktur z.B. eines Körperimplantat zu ermöglichen.

Diese Aufgabe wird durch die Merkmale der unabhängigen Ansprüche gelöst. Vorteilhafte Weiterbildungen der Erfindung sind in den abhängigen Ansprüchen definiert.

Gemäß einem Gesichtspunkt wird ein Verfahren zum Herstellen eines Körperimplantats mit folgenden Schritten zur Verfügung gestellt:
Erzeugen einer Textilstruktur oder eines Geflechts aus einem ersten Fasermaterial bzw. Draht bzw. Garn;
Durchführen einer ersten Formgebung an der Textilstruktur bzw. dem Geflecht beispielsweise durch eine Wärmebehandlung;
Entfernen eines Teils der Fasern des ersten Fasermaterial, und
Ersetzen durch ein zweites Fasermaterial bzw. Draht bzw. Garn oder Wiedereinsetzen des ersten Fasermaterials nach Durchführung eines weiteren Bearbeitungsschritts an dem entfernten Teil der Fasern des ersten Fasermaterials.

Indem an der fertigen Textilstruktur bzw. dem fertigen Geflecht ein Teil der Fasern bzw. der Drähte oder des Garns entfernt wird, kann dieser Teil durch ein zweites unterschiedliches Fasermaterial mit unterschiedlichen Eigenschaften ersetzt werden. Alternativ oder zusätzlich kann auch das erste Fasermaterial nach der Durchführung eines weiteren Bearbeitungsschritts wie beispielsweise Elektropolieren und/oder Beschichtung wieder eingesetzt werden. Das Entfernen des Teils der Fasern des ersten Materials kann beispielsweise durch Rückwärtsflechten erfolgen, beispielsweise durch Betreiben der Flechtmaschine in der umgekehrten Richtung, um einen Teil der bereits in das Geflecht eingebundenen Fasern wieder aus dem Geflecht zu entfernen.

Vorzugsweise folgt nach dem Schritt "Ersetzen durch ein zweites Fasermaterial bzw. Draht bzw. Garn oder Wiedereinsetzen des ersten Fasermaterials nach Durchführung eines weiteren Bearbeitungsschritts an dem entfernten Teil der Fasern des ersten Fasermaterials" ein Schritt des (Wieder-)Erzeugens der Textilstruktur bzw. einer (neuen) Textilstruktur.

Gemäß einem weiteren Gesichtspunkt wird ein Verfahren zum Herstellen eines Körperimplantats mit folgenden Schritten zur Verfügung gestellt:
Erzeugen einer (ersten) Textilstruktur oder eines Geflechts aus einem ersten Fasermaterial bzw. Draht bzw. Garn;
Durchführen einer ersten Formgebung an der (ersten) Textilstruktur bzw. dem Geflecht beispielsweise durch eine Wärmebehandlung;
Öffnen der (ersten) Textilstruktur aus dem ersten Fasermaterial;
Bereitstellen mindestens eines zweiten Fasermaterials; und
Herstellen einer (zweiten, abgewandelten) Textilstruktur aus dem ersten Fasermaterial kombiniert mit dem mindestens zweiten Fasermaterial.

Unter dem Begriff "öffnen" ist insbesondere zu verstehen, dass das Geflecht wieder entflochten wird. Ein "Öffnen" kann als Umkehrung bzw. inverser Vorgang zum Erzeugen einer Textilstruktur oder eines Geflechts verstanden werden. Beispielsweise kann eine Textilstruktur/Geflecht durch Rückwärtsflechten geöffnet werden.

Vorzugsweise wird bei einem Öffnen das erste Fasermaterial "in Position gehalten". Beispielsweise können Enden des ersten Fasermaterials (weiterhin) mittels Haltevorrichtungen wie Greifern oder Draht fixiert bleiben, so dass lediglich der vorherige Flechtvorgang, nämlich das Erzeugen der ersten Textilstruktur/ Geflecht umgekehrt bzw. rückgängig gemacht wird. Im Weiteren kann dann ein zweites Fasermaterial oder weitere Fasermaterialien "hinzugefügt" werden, so dass dann eine (neue, zweite, abgewandelte) Textilstruktur erzeugt werden kann, die sowohl erstes Fasermaterial als auch zweites und ggf. weiteres Fasermaterial umfasst. Zusätzlich können in einem Schritt Teile/ Fasern des ersten Fasermaterials vor oder nach einem Öffnen der (ersten) Textilstruktur entfernt werden.

Gemäß der obigen Verfahren kann nach dem Einbinden des zweiten Fasermaterials die Textilstruktur einer zweiten Formgebung bzw. Wärmebehandlung unterzogen werden, die so konfiguriert ist, dass bei der zweiten Formgebung nur das zweite Fasermaterial eine Formgebung (Shape Setting) erhält. Dies wird beispielsweise dadurch erzielt, dass eine zweite Wärmebehandlung bei einer niedrigeren Temperatur und/oder mit kürzerer Einwirkungsdauer als die erste Wärmebehandlung durchgeführt wird. Die zweite Wärmebehandlung führt somit zu einer Formgebung des zweiten Fasermaterials, ohne die bei der ersten Wärmebehandlung an dem ersten Fasermaterial durchgeführte Formgebung zu beeinflussen. In dem Fall, dass es nicht möglich ist, die Formgebung des ersten Fasermaterials durch Durchführen der zweiten Wärmebehandlung nicht zu beeinflussen, beispielsweise in dem Fall, wenn die Temperaturen der ersten und zweiten Wärmebehandlung zu nahe beieinander liegen, kann die zweite Wärmebehandlung an dem zweiten Fasermaterial auch separat vor dem Einsetzen des zweiten Fasermaterials in die Textilstruktur erfolgen.

Vorzugsweise weist das Verfahren des Weiteren den Schritt des Durchführens einer zweiten Formgebung bzw. Wärmebehandlung an dem zweiten Fasermaterial auf, wobei die zweite Formgebung bzw. Wärmebehandlung so konfiguriert ist, dass nur das zweite Fasermaterial eine Formgebung erhält.

Vorzugsweise wird eine zweite Wärmebehandlung bei einer niedrigeren Temperatur als die erste Wärmebehandlung durchgeführt.

Dabei dient die Wärmebehandlung einer Formgebung der Textilstruktur bzw. des Geflechts. Das der Textilverarbeitung unterzogene Fasermaterial wird beispielsweise über einen vorgegebenen Zeitraum erwärmt, um einen Formgebungsschritt durchzuführen. Dabei werden beispielsweise durch die Textilverarbeitung entstandene Spannungen im Fasermaterial abgebaut oder im Wesentlichen eliminiert.

Ein weiteres Beispiel kann ein Formgedächtniseffekt sein, wie ihn viele Materialien, u.a. Nitinol aufweisen. Dies bedeutet, dass zunächst ein Metallgefüge in einer sogenannten Martensit-Struktur vorliegt, das durch Erhöhen der Temperatur durch Phasenumwandlung in eine Austenit-Struktur umgewandelt wird. Außergewöhnlich bei Nitinol ist, dass diese Umwandlung reversibel ist, ohne dass plastische Defekte auftreten. Die Phasenumwandlung findet diffusionslos statt, d.h. ohne dass die Atome ihre Plätze in der Gitterstruktur wechseln.

Insbesondere nehmen bei dieser diffusionslosen, reversiblen Phasenumwandlung der Austenit- in die Martensitstruktur die Atome durch reine Scherdeformation eine geordnete Zwillingsanordnung ein.

Weiter bevorzugt wird die zweite Formgebung bzw. Wärmebehandlung an der aus den zwei Fasermaterialien erzeugten Textilstruktur durchgeführt oder vor dem Einsetzen des zweiten Fasermaterials separat durchgeführt.

Vorzugsweise wird die Textilstruktur durch Textilverarbeitung, Flechten, Weben, Wirken oder Stricken erzeugt.

Weiter bevorzugt kann die Textilstruktur bzw. das Geflecht zumindest ein drittes Fasermaterial aufweisen.

Vorzugsweise wird zumindest eines der Fasermaterialien vor dem endgültigen Einbinden in die Textilstruktur bzw. das Geflecht elektropoliert und/oder beschichtet. Dieser Schritt kann auch nach dem Entfernen eines Teils des ersten Fasermaterials erfolgen. In anderen Worten wird zunächst eine Textilstruktur erzeugt, dann ein erster Formgebungsschritt durch eine erste Wärmebehandlung durchgeführt und nach dem Entfernen eines Teils der Fasern des ersten Fasermaterials dieser Teil des ersten Fasermaterials elektropoliert und/oder beschichtet und anschließend wieder in die Textilstruktur eingebunden.

Vorzugsweise weist das erste Fasermaterial Nitinol auf und das zweite oder dritte Fasermaterial eines aus einem Polymer, einem bioabbaubaren Polymer und Wolfram. Nitinol eignet sich insbesondere zum Erzeugen von Formgedächtniseigenschaften, um beispielsweise ein komprimiertes Körperimplantat wie beispielsweise einen Stent nach dem Plazieren im Körper aufgrund der Formgedächtniseigenschaften zu expandieren. Wolfram kann beispielsweise die Röntgensichtbarkeit eines Körperimplantats erhöhen.

Weiter bevorzugt weist zumindest eines der Fasermaterialien Formgedächtniseigenschaften auf.

Gemäß der obigen Verfahren kann Fasermaterial insbesondere eine oder mehrere Fasern und/oder einen oder mehrere Drähte und/oder ein oder mehrere Garne umfassen. In anderen Worten kann ein erstes Fasermaterial aus einem oder mehreren Fasern gebildet werden. Zusätzlich oder alternativ kann ein erstes Fasermaterial aus einem oder mehreren Garnen gebildet werden. Zusätzlich oder alternativ kann ein erstes Fasermaterial aus einem oder mehreren Drähten gebildet werden. Analoges ist ebenfalls zutreffend für zweites, drittes, viertes, etc. Fasermaterial.

Vorteilhafterweise kann das zweite und/oder weitere Fasermaterial als Hilfswerkstoff dienen, um z.B. eine höhere Dichtigkeit zu erzielen. Insbesondere können mehr als zwei Fasermaterialien kombiniert werden. Beispielsweise kann neben einem zweiten auch ein drittes oder viertes Fasermaterial kombiniert bzw. verflochten werden. In anderen Worten können weitere Fasermaterialien mit dem ersten und zweiten Fasermaterial kombiniert werden, wobei die Fasermaterialien voneinander verschiedene Eigenschaften (Festigkeit, Zähigkeit, Steifigkeit, Bioresorbierbarkeit, etc.) aufweisen können.

Vorzugsweise kann mit den vorgenannten aufgezeigten Verfahren eine Textilstruktur hergestellt werden, die eine Formkombination aufweist, indem zwei oder mehrere Textilstrukturen miteinander verbunden werden. Dabei ist es möglich, unterschiedliche Materialien, aber auch gleiche Materialien mit gleichen oder unterschiedlichen vorangegangenen Bearbeitungsschritten zu kombinieren. Unter "unterschiedlichen vorangegangenen Bearbeitungsschritten" ist beispielsweise zu verstehen, dass die Fasermaterialien unterschiedlichen Temperaturbehandlungen unterzogen werden/wurden. Beispielsweise kann ein erstes und ein zweites Fasermaterial aus dem gleichen Werkstoff wie beispielsweise Nitinol hergestellt sein, jedoch das erste Fasermaterial einer anderen von dem zweiten Fasermaterial verschiedenen Wärmebehandlung unterzogen worden sein/werden.

In einer weiteren Ausgestaltung kann das erste Fasermaterial verschiedene Werkstoffe oder Behandlungen umfassen. Beispielsweise kann das erste Fasermaterial Polymerfäden/-garne sowie Nitinolfäden/-drähte umfassen. Ein zweite Fasermaterial, welches mit dem ersten Fasermaterial kombiniert wird, weist in diesem Fall vorzugsweise andere material- und/oder behandlungsbedinge Eigenschaften auf.

Vorteilhafterweise erlauben die obigen Verfahren das flexible Gestalten von Textilstrukturen. Unter anderem kann gemäß der obigen Verfahren ein später eingebundenes Fasermaterial eine (Haupt-)Struktur verlassen, um eine eigene Folgestruktur bereitzustellen bzw. zu bilden. Beispielsweise kann mit der Kombination aus ersten und zweiten Fasermaterial eine Textilstruktur gebildet werden, wobei (nur) das zweite Fasermaterial aus dieser Textilstruktur herausgeführt bzw. weitergeführt wird, um eine eigene Textilstruktur/Folgestruktur zu bilden. Weiterhin beispielsweise kann (nur) ein drittes Fasermaterial aus der (Haupt-) Textilstruktur herausgeführt werden, um eine Folgestruktur zu bilden, während das erste und zweite Fasermaterial nicht in der Folgestruktur weitergeführt werden.

Insbesondere sind durch das zur Verfügung gestellte Verfahren Filterbauteile oder vergleichbare Bauteile mit einer äußeren starren Struktur zur Verankerung am Gefäß und einem flexibleren inneren Teil herstellbar.

Vorzugsweise ist das erste Fasermaterial ein wärmebehandelbares Metall oder ein wärmebehandelbares Nichtmetall.

Vorzugsweise ist das zweite Fasermaterial aus Metall und/oder Nichtmetall. Weiterhin vorzugsweise ist das dritte Fasermaterial oder weitere Fasermaterial aus Metall und/oder Nichtmetall.

Vorzugsweise ist das zweite Fasermaterial aus beschichtetem Garn oder beschichtetem Draht. Weiterhin vorzugsweise sind das dritte und ggf. weitere Fasermaterialien aus beschichtetem Garn oder beschichtetem Draht. Vorzugsweise können das zweite und das/die weitere(n) Fasermaterial(ien) verschiedene Garne oder Drähte und/oder Beschichtungen aufweisen.

Vorzugsweise können das zweite und das/die weitere(n) Fasermaterial(ien) verschiedene Garne/Draht und/oder Beschichtungen aufweisen.

Weiterhin vorzugsweise kann vorgesehen sein, dass die ersten Fasermaterialen in der Textilstruktur verbleiben und ein mindestens zweites Fasermaterial eingefügt wird.

Vorzugsweise ist das zweite Fasermaterial aus demselben Werkstoff wie das erste Fasermaterial, wobei das zweite Fasermaterial einer von der dem ersten Fasermaterial verschiedenen Formgebung unterzogen wurde. Analoges trifft auch auf drittes, viertes, etc. Fasermaterial zu.

Vorzugsweise umfasst das zweite Fasermaterial einen von dem ersten Fasermaterial verschiedenen, zweiten Werkstoff. Zusätzlich vorzugsweise wird das zweite Fasermaterial von der dem ersten Fasermaterial verschiedenen Formgebung unterzogen. Analoges trifft auch auf drittes, viertes, etc. Fasermaterial zu.

Vorzugsweise umfasst das zweite Fasermaterial denselben Werkstoff wie das erste Fasermaterial, wobei das zweite Fasermaterial aber von dem ersten Fasermaterial verschiedene Eigenschaften aufweist und/oder unterschiedlich bearbeitet wurde. Beispielsweise können das erste Fasermaterial und das zweite Fasermaterial mit voneinander verschiedenen Wärmbehandlungen wie beispielsweise einem Austenitfinish (Af) behandelt werden/worden sein. Weiterhin können das erste und zweite Fasermaterial voneinander verschiedene Beschichtungen, wie Vergoldung, PTFE-Beschichtung etc, aufweisen. Analoges trifft auch auf drittes, viertes, etc. Fasermaterial zu.

Weiterhin vorzugsweise kann das zweite und/oder weiteres Fasermaterial als Verbindungselement bzw. Verbindungsmaterial zwischen zwei Textilstrukturen . eingefügt/eingesetzt werden. Analoges trifft auch auf drittes, viertes, etc. Fasermaterial zu. In anderen Worten kann das zweite Fasermaterial und/oder ein drittes, viertes, etc. Fasermaterial ein Verbindungselement zwischen zumindest zwei Textilstrukturen bilden.

Durch das vorstehend beschriebene Verfahren kann ein Körperimplantat wie beispielsweise einen Stent, ein Stentgraft, eine Gefäßstütze, ein Herzklappenrahmen oder dergleichen hergestellt werden, wobei das Körperimplantat eine Textilstruktur oder ein Geflecht aus einem ersten Fasermaterial und einem zweiten Fasermaterial aufweist, wobei zumindest an dem ersten Fasermaterial eine erste Wärmebehandlung zum Erzeugen einer Formgebung (Shape Setting) durchgeführt ist.

Vorzugsweise hat das Körperimplantat zumindest zwei unterschiedliche Fasermaterialien. Beispielsweise kann das eine Fasermaterial Nitinol mit Formgedächtniseigenschaften sein und das andere Fasermaterial ein Material mit guter Röntgensichtbarkeit, wie beispielsweise Tantal oder Gold.

Das Körperimplantat kann darüber hinaus auch ein drittes und weitere unterschiedliche Fasermaterialien aufweisen. Diese können Polymere, bioabbaubare Polymere, Wolfram oder dergleichen aufweisen.

Vorzugsweise ist eines der obigen Körperimplantate eine Gefäßprothese.

Vorzugsweise ist eines der obigen Körperimplantate eine Stentgraft.

Vorzugsweise ist eines der obigen Körperimplantate ein Okkluder.

Vorzugsweise ist eines der obigen Körperimplantate ein Flow Diverter.

Vorzugsweise ist eines der obigen Körperimplantate ein Aneurysma Coil

Vorzugsweise ist eines der obigen Körperimplantate ein Herzklappenrahmen.

Vorzugsweise ist eines der obigen Körperimplantate ein Abdominales Aorten Aneurysma (AAA-) Stent.

Vorzugsweise ist eines der obigen Körperimplantate ein Thorakales Aorten Aneurysma (TAA-)Stent.

Vorzugsweise ist eines der obigen Körperimplantate ein gastroenterologischer Stent.

Vorzugsweise ist eines der obigen Körperimplantate ein peripherer Stent.

Die Erfindung wird nun anhand der beigefügten Zeichnungen und bevorzugter Ausführungsbeispiele näher erläutert.
Fig. 1 zeigt im Schritt a) das Erzeugen eines Geflechts mit einem ersten Fasermaterial und im Schritt b) das Shape Setting nach dem Durchführen der ersten Wärmebehandlung an dem Geflecht sowie im Schritt c) das Ersetzen eines Teils des ersten Fasermaterials durch ein zweites Fasermaterial.
Fig. 2 zeigt das Erzeugen eines Geflechts mit dem ersten Fasermaterial mittels einer Flechtmaschine.
Fig. 3 zeigt das Rückwärtslaufen einer Flechtmaschine zum Entfernen eines Teils des ersten Fasermaterials, an dem zuvor eine Wärmebehandlung für die Formgebung durchgeführt wurde.
Fig. 4 zeigt einzelne Fasern eines ersten und eines zweiten Fasermaterials, wobei an dem ersten Fasermaterial eine Formgebung durchgeführt wurde.
Fig. 5 zeigt das Ersetzen des entfernten Teiles des ersten Fasermaterials durch ein zweites Fasermaterial.
Fig. 6 zeigt im Schritt a) das Erzeugen eines Geflechts mit einem ersten Fasermaterial und im Schritt b) das Durchführen einer ersten Wärmebehandlung an dem Geflecht aus dem ersten Fasermaterial;
   in Fig. 6d) wird das Erzeugen eines Geflechts mit einem zweiten Fasermaterial gezeigt und in Fig. 6c) das Durchführen einer zweiten Wärmebehandlung an dem Geflecht aus dem zweiten Fasermaterial; und
   in Fig. 6e) ist das Zusammenführen des ersten und zweiten Fasermaterials in einem Geflecht gezeigt.
Fig. 7 zeigt schematisch das Herstellen einer Textilstruktur aus einem ersten Fasermaterial kombiniert mit mindestens einem zweiten Fasermaterial.
Fig. 8 zeigt eine Prinzipskizze einer Textilstruktur mit einer Formkombination, indem zwei oder mehrere Textilstrukturen miteinander verbunden werden.
Fig. 9 zeigt eine Prinzipskizze einer Textilstruktur, bei der das zweite Fasermaterial die ursprüngliche Textilstruktur verlässt und eine eigene Struktur/Form bildet.

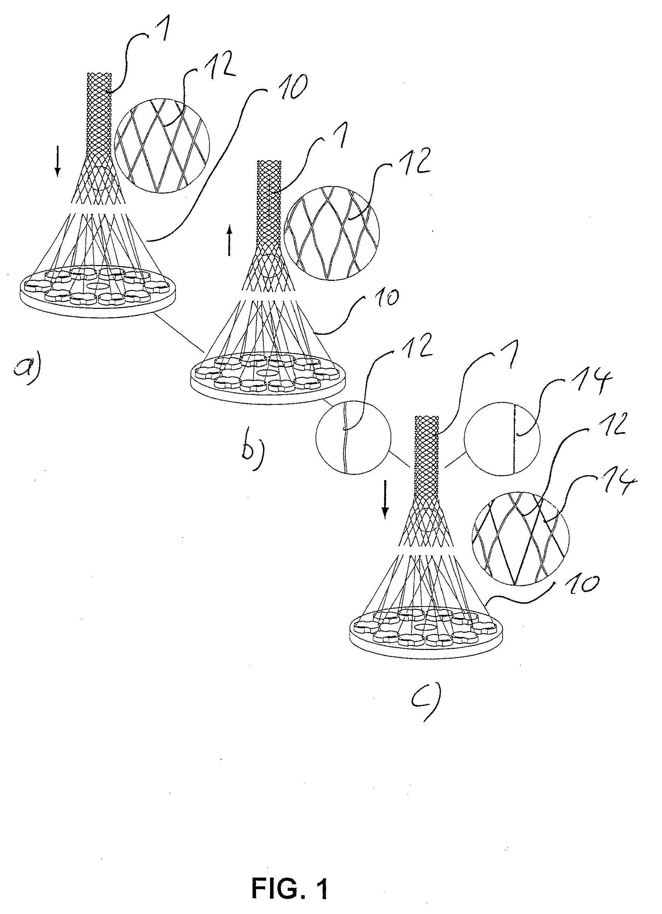

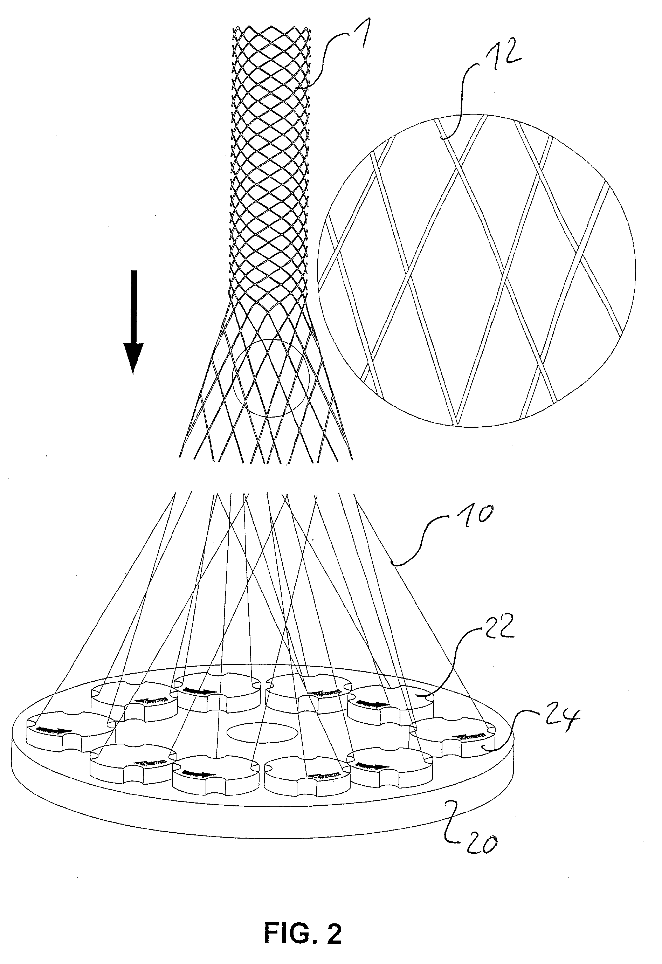

Wie in Fig. 1a und Fig. 2 gezeigt ist, wird gemäß einem bevorzugten Ausführungsbeispiel zunächst mit einer Flechtmaschine 20, die erste Klöppel 22 und zweite Klöppel 24 aufweist, ein Geflecht als Textilstruktur 10 mit einem ersten Fasermaterial 12 erzeugt. Hierzu drehen sich die ersten Klöppel 22 entgegen dem Uhrzeigersinn und die zweiten Klöppel 24 drehen sich im Uhrzeigersinn. Aus der Textilstruktur 10 bzw. dem Geflecht kann ein Körperimplantat 1 in einer rohrförmigen Gestalt erzeugt werden.

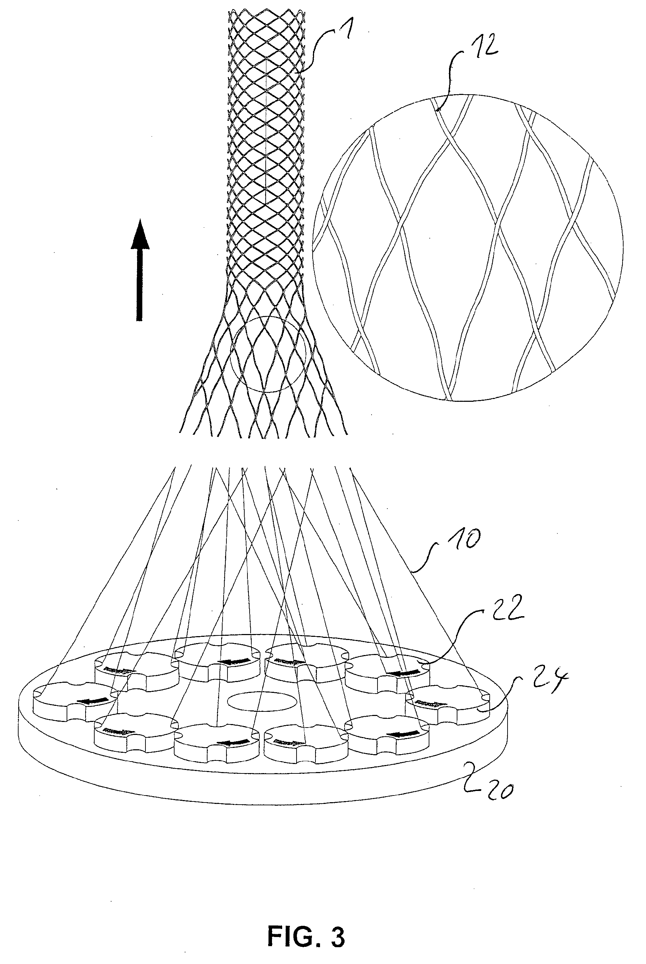

Wie in Figur 3 gezeigt ist, wird an der Textilstruktur 10 bzw. dem Geflecht eine erste Wärmebehandlung zum Erzeugen einer Formgebung des ersten Fasermaterials 12 durchgeführt. Auf diese Weise erhält das erste Fasermaterial 12, beispielsweise Nitinol, eine Formgebung mit Formgedächtniseigenschaften, um beispielsweise eine Expansion nach dem Einsetzen des Körperimplantats 1 in einen lebenden Körper zu bewirken.

Dabei dient die Wärmebehandlung einer Formgebung des Geflechts 10. Das der Textilverarbeitung unterzogene erste Fasermaterial 12 wird beispielsweise über einen vorgegebenen Zeitraum erwärmt, um einen Formgebungsschritt durchzuführen. Dabei werden beispielsweise durch die Textilverarbeitung entstandene Spannungen im ersten Fasermaterial 12 abgebaut oder im wesentlichen eliminiert.

Ein weiteres Beispiel kann ein Formgedächtniseffekt sein, wie ihn viele Materialien, u.a. Nitinol aufweisen. Dies bedeutet, dass zunächst ein Metallgefüge in einer sogenannten Martensit-Struktur in dem ersten Fasermaterial 12 vorliegt, das durch Erhöhen der Temperatur durch Phasenumwandlung in eine Austenit-Struktur umgewandelt wird. Außergewöhnlich bei Nitinol ist, dass diese Umwandlung reversibel ist, ohne dass plastische Defekte auftreten. Die Phasenumwandlung findet somit diffusionslos statt, d.h. ohne dass die Atome ihre Plätze in der Gitterstruktur wechseln.

Insbesondere nehmen bei dieser diffusionslosen, reversiblen Phasenumwandlung der Austenit- in die Martensitstruktur die Atome durch reine Scherdeformation eine geordnete Zwillingsanordnung ein.

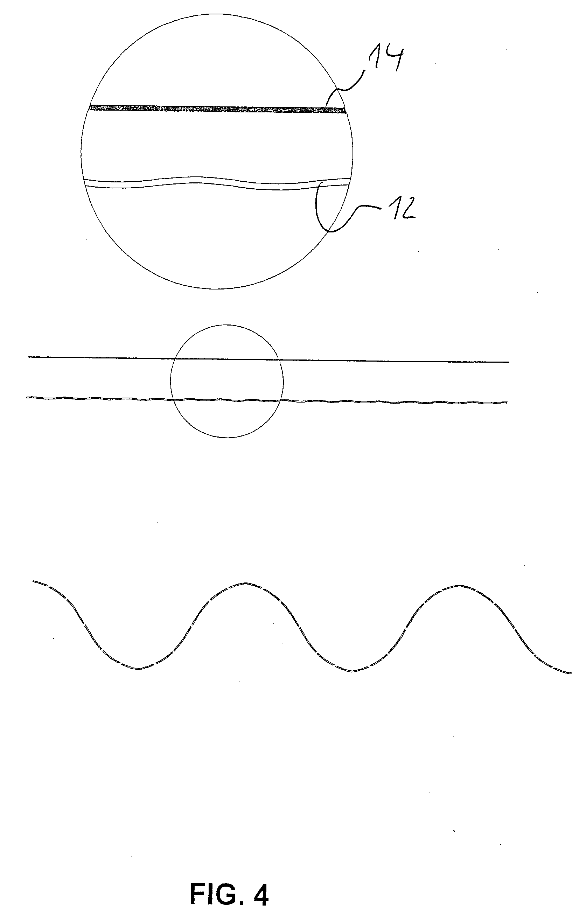

Fig. 4 zeigt das erste Fasermaterial 12 mit der durchgeführten Formgebung aufgrund des ersten Wärmebehandlungsschritts sowie ein zweites Fasermaterial 14, das noch keine Wärmebehandlung erfahren hat. Demgemäß hat das erste Fasermaterial 12 beispielsweise eine wellenartige Form, während das zweite Fasermaterial 14 eine gerade Draht- oder Garnform hat.

Wie in Fig. 3 gezeigt ist, kann ein Teil des ersten Fasermaterials 12 nach dem Formgebungsschritt aus dem Geflecht 10 entfernt werden, indem die Flechtmaschine 12 rückwärts betrieben wird, d.h. die ersten Klöppel 22, die sich im Normalbetrieb entgegen dem Uhrzeigersinn drehen, sich nunmehr im Uhrzeigersinn drehen, und die zweiten Klöppel 24, die sich im Normalbetrieb im Uhrzeigersinn drehen, nunmehr entgegen dem Uhrzeigersinn gedreht werden. Auf diese Weise wird das fertige Geflecht 10 bestehend aus dem mit dem Formgebungsschritt versehenen ersten Fasermaterial 12 teilweise geöffnet.

In das teilweise geöffnete Geflecht 10 kann nun entweder das erste Fasermaterial 12 nach dem Durchführen eines weiteren Behandlungsschritts wieder eingeführt werden, beispielsweise kann das erste Fasermaterial 12 als weiteren Behandlungsschritt an Knotenpunkten elektropoliert oder beschichtet werden.

Dies ist innerhalb des Geflechts 10 nicht oder nur unter erschwerten Bedingungen möglich. Durch Entfernen des Teils des ersten Fasermaterials 12 kann dieses separat von dem Geflecht 10 elektropoliert und/oder beschichtet werden, um eine hohe Qualität aufzuweisen. Danach wird der Teil des ersten Fasermaterials 12 durch Flechten wieder in das Geflecht 10 eingebunden.

Der weitere Behandlungsschritt kann auch ein sogenanntes "drug-eluting" beinhalten, d.h. ein Beschichten mit einem Medikament, indem das erste Fasermaterial 12 beispielsweise in eine Lösung des Medikaments eingetaucht oder damit besprüht wird. Das auf diese Weise auf das Körperimplantat 1 aufgebrachte Medikament kann nach dem Einführen in einen menschlichen Körper dort sukzessive abgegeben werden.

Beispielsweise ein sogenannter drug eluting stent (DES) als Körperimplantat 1 kann kleine Mengen von Arzneistoffen freisetzen, die die Zellneubildung hemmen. Zwei Wirkstoffe haben sich bei der Behandlung mit medikamentenfreisetzenden Stents durchgesetzt: das Immunsuppressivum Sirolimus und das Krebstherapeutikum Paclitaxel. Solche Stents können beispielsweise zur Therapie der koronaren Herzkrankheit eingesetzt werden.

Alternativ kann auch anstelle des entfernten Teils des ersten Fasermaterials 12 oder eines Teils davon ein zweites Fasermaterial 14 mit unterschiedlichen Eigenschaften eingebunden werden. Beispielsweise kann, wenn das erste Fasermaterial 12 Nitinol aufweist, das zweite Fasermaterial ein Polymer, ein bioabbaubares Polymer und/oder Wolfram aufweisen.

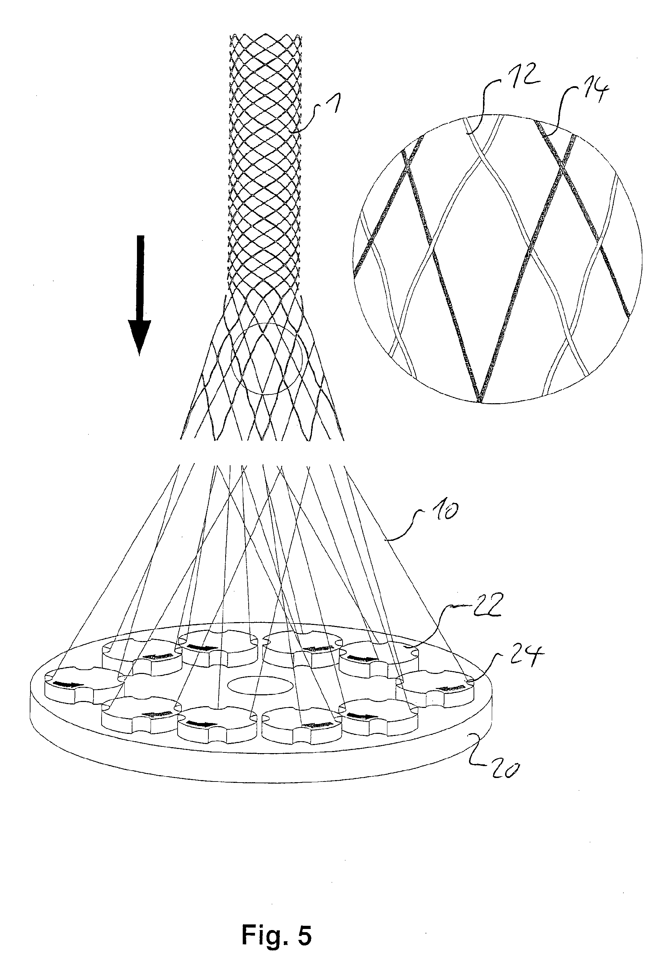

Nach dem Einbinden des zweiten Fasermaterials 14, hat das Geflecht 10 sowohl Fasern aus dem ersten Fasermaterial 12 sowie Fasern aus dem zweiten Fasermaterial 14, wie in Fig. 5 gezeigt ist.

Danach kann an dem fertigen Geflecht 10 bestehend aus dem ersten Fasermaterial 12 mit der bereits durchgeführten Formgebung sowie dem zweiten Fasermaterial 14 ohne Formgebung ein zweiter Formgebungsschritt durch Durchführen einer zweiten Wärmebehandlung an dem fertigen Geflecht 10 durchgeführt werden. Dabei sollte die zweite Wärmebehandlung so konfiguriert sein, dass die Formgebung des ersten Fasermaterials 12 im Wesentlichen nicht beeinflußt wird. Dies wird insbesondere dadurch erzielt, dass die zweite Wärmebehandlung bei einer niedrigeren Temperatur als die erste Wärmebehandlung und/oder über eine geringere Zeitdauer als die erste Wärmebehandlung durchgeführt wird.

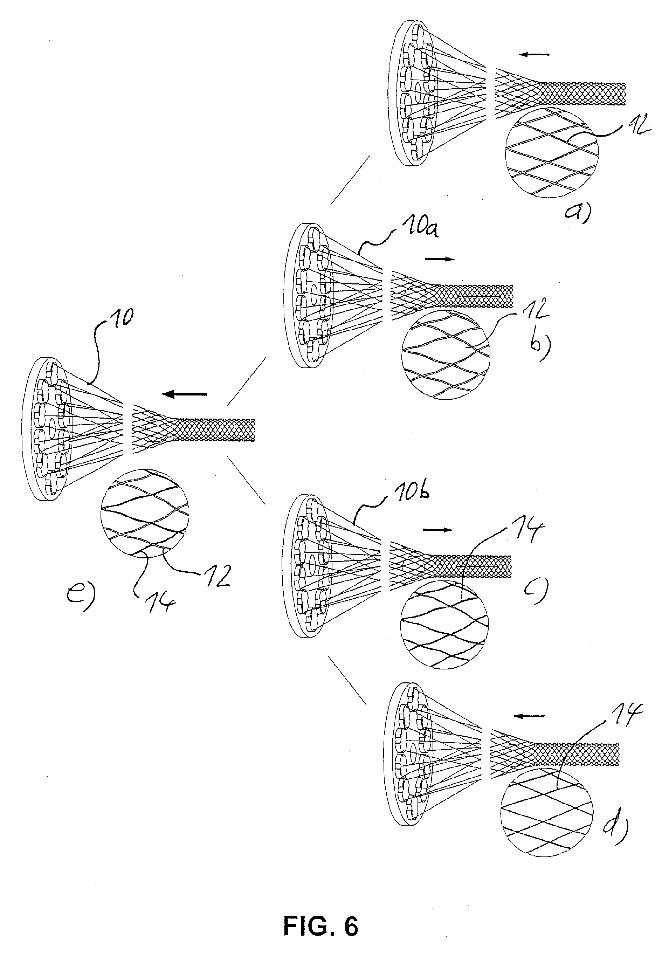

Sollten die Temperaturen der ersten und zweiten Wärmebehandlung des ersten und zweiten Fasermaterials 12, 14 zu nahe beieinander liegen, so dass eine unerwünschte Beeinflussung der Formgebung des ersten Fasermaterials 12 durch die zweite Wärmebehandlung nicht vermieden werden kann, können die Wärmebehandlungen auch separat durchgeführt werden, wie in Fig. 6 gezeigt ist. Bei diesem Verfahren wird ein Geflecht 10a aus dem ersten Fasermaterial 12 erzeugt, wie in Schritt a) gezeigt ist und eine erste Wärmebehandlung an dem ersten Fasermaterial 12 durchgeführt, wie in Schritt b) gezeigt ist. Dasselbe erfolgt separat mit dem zweiten Fasermaterial 14, das heißt es wird ein Geflecht 10b aus dem zweiten Fasermaterial 14 erzeugt, wie in Schritt d) gezeigt ist und es wird eine zweite Wärmebehandlung an dem aus dem zweiten Fasermaterial 14 erzeugten Geflecht 10b durchgeführt, wie in Schritt c) gezeigt ist.

Danach werden die beiden Geflechte 10a, 10b zu einem Geflecht 10 zusammengeführt, wie in Schritt e) von Fig. 6 gezeigt ist. Dies kann beispielsweise dadurch erfolgen, dass eines der zuvor erzeugten Geflechte 10a, 10b aus den Schritten a, b bzw. c, d vollständig geöffnet bzw. aufgelöst wird und in das jeweils andere Geflecht 10b, 10a eingeflochten wird oder ein Teil eines Geflechts 10a entfernt wird und dieser Teil durch das Fasermaterial 14 des anderen Geflechts 10b ersetzt wird.

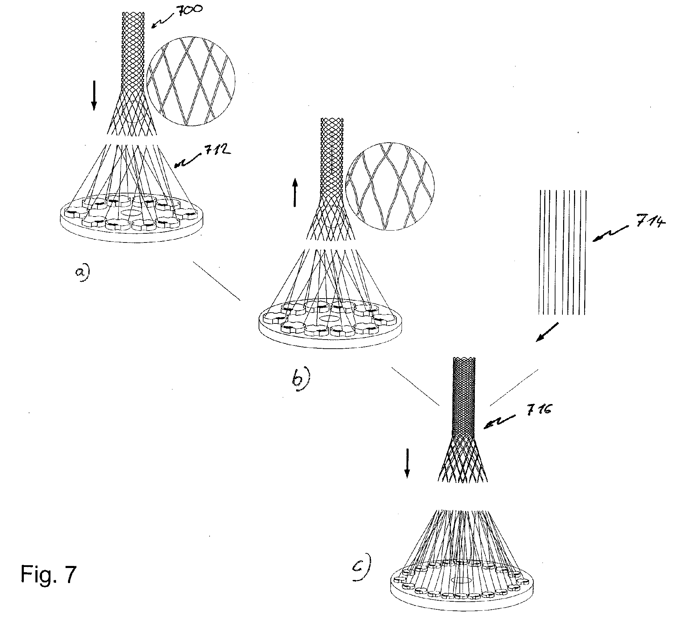

Fig. 7 zeigt schematisch das Herstellen einer Textilstruktur aus einem ersten Fasermaterial kombiniert mit mindestens einem zweiten Fasermaterial.

Insbesondere zeigt Figur 7 im Schritt a) das Erzeugen einer Textilstruktur 700 oder eines Geflechts 700 aus einem ersten Fasermaterial 712, und im Schritt b) das Durchführen einer ersten Formgebung an der Textilstruktur oder dem Geflecht beispielsweise durch eine Wärmebehandlung sowie im Schritt c) das Herstellen einer abgewandelten Textilstruktur 716 aus dem ersten Fasermaterial 712 kombiniert mit dem mindestens zweiten Fasermaterial 714.

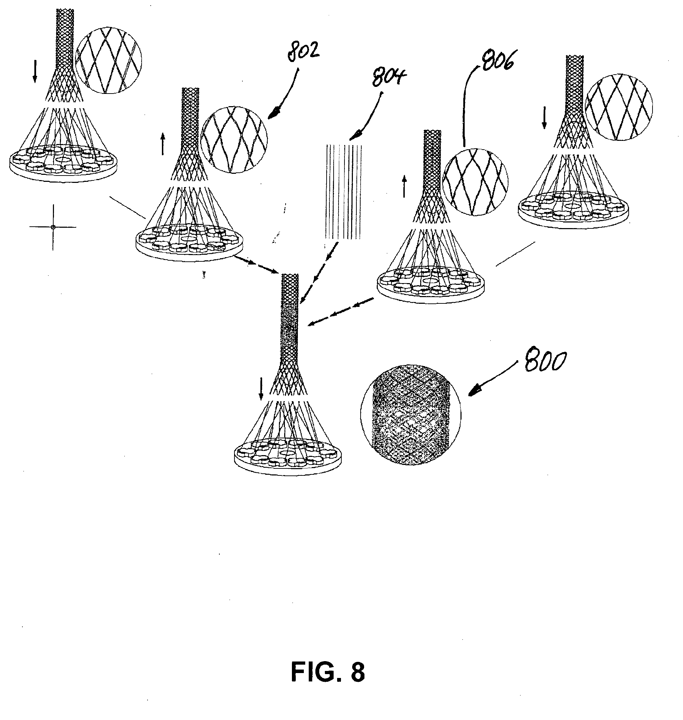

Fig. 8 zeigt eine Prinzipskizze einer Textilstruktur mit einer Formkombination 800, indem zwei oder mehrere Textilstrukturen miteinander verbunden werden. Insbesondere zeigt Figur 8 eine erste Textilstruktur 802, die mit einer zweiten Textilstruktur 804 verbunden ist. Die zweite Textilstruktur 804 ist wiederum mit einer dritten Textilstruktur 806 verbunden. Diese drei miteinander verbundenen bzw. ineinander übergehenden Textilstrukturen bilden die Formkombination 800.

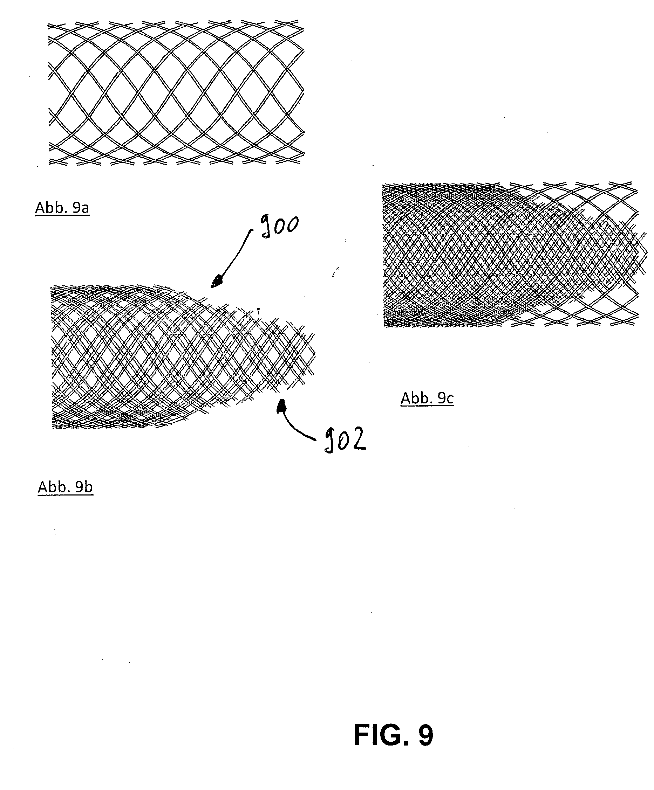

Fig. 9 zeigt eine Prinzipskizze einer Textilstruktur, bei der das zweite Fasermaterial die ursprüngliche Textilstruktur verlässt und eine eigene Struktur/Form bildet.

Wie aus Figur 9 ersichtlich ist, können Textilstrukturen von flexibler Gestalt bereitgestellt werden. Figur 9 zeigt eine Hauptstruktur 900 später eingebundenes Fasermaterial die Hauptstruktur 900 verlassen, um eine eigene Folgestruktur 902 bereitzustellen bzw. zu bilden. Beispielsweise kann mit der Kombination aus ersten und zweiten Fasermaterial eine Textilstruktur gebildet werden, wobei (nur) das zweite Fasermaterial aus dieser Textilstruktur herausgeführt bzw. weitergeführt wird, um eine eigene Textilstruktur/Folgestruktur zu bilden. Beispielsweise kann die Hauptstruktur 900 einer Textilstruktur 716 entsprechen, wobei in einem weiteren Herstellungsschritt bspw. Flechten die Folgestruktur hergestellt wird.

Wie vorstehend erläutert ist, kann durch das neuartige Verfahren ein Geflecht 10 zum Erzeugen eines Körperimplantats 1, wie beispielsweise einer Herzklappe, eines Stents oder einer Gefäßstütze erzeugt werden, das aus zwei unterschiedlichen Fasermaterialien 12, 14 besteht. Es kann darüber hinaus auch mindestens ein (nicht gezeigtes) drittes Fasermaterial in das Geflecht 10 des Körperimplantats 1 eingeflochten werden. Dabei können unterschiedliche Wärmebehandlungen an den unterschiedlichen Fasermaterialien 12, 14 durchgeführt werden, um eine Formgebung (Shape Setting) zu erzeugen. Auf diese Weise kann das Geflecht 10 vorgegebene Formgedächtniseigenschaften erhalten und gleichzeitig vorteilhafte Eigenschaften, beispielsweise in Bezug auf eine Biokompatibilität, Bioabbaufähigkeit, oder Röntgensichtbarkeit, aufzuweisen. Es können beispielsweise Marker aus einem röntgensichtbarem Material auf vorteilhafte Weise in das Geflecht 10 eingebunden werden.

Die Erfindung ist nicht auf ein Flechten beschränkt, sondern die Textilstruktur 10 kann auch durch eine Textilverarbeitung, Weben, Wirken oder Stricken erzeugt werden. Das Fasermaterial 12, 14 kann auch ein Draht oder ein Garn sein. Die Erfindung ist des weiteren nicht auf die Durchführung auf einer Flechtmaschine 20 beschränkt, sondern kann auch durch jede andere Textilverarbeitungsmaschine durchgeführt werden, die eine entsprechende Textilstruktur erzeugen kann. Das Verfahren ist nicht zwingend auf Körperimplantate beschränkt, sondern kann auf alle Anwendungsbereiche ausgedehnt werden, bei denen es erforderlich ist, eine Textilstruktur mit zwei Materialien, die wärmebehandelt sein müssen (min. ein Material), aber nicht gleichzeitig wärmebehandelt werden können, zu realisieren.

Vorteilhafterweise können durch ein erfindungsgemäß gebildetes Körperimplantat, wenn es in ein Gefäß eingesetzt wird, die Blutströmung durch das Gefäß verbessert werden. Eingebrachte Fasern können unter anderem das Strömungsverhalten beeinflussen. Dieses wird z.B. bei den sogenannten Flow Divertern genutzt, bei dem der Blutstrom abgelenkt wird und beispielsweise ein Aneurysma vom Blutstrom abschneidet.

Beim Einsatz von Multifilamenten wird die Oberfläche deutlich vergrößert. Es kann sich als vorteilhaft erweisen, die Thrombogenität zur Bildung von Verschlüssen, z.B. Occluder für LAA (Left Atrial Appendage) oder PFO (Patent Foramen Ovale), zu erhöhen.
Bei geeigneter Oberflächeneigenschaft könnte die Bildung von Epithelzellen gefördert werden. Wird ein degradierbares Material eingesetzt kann das verbleibende metallische Material auf ein Minimum reduziert werden. Dies könnte sich positiv auf die Zahl der Wiederverschlüsse, sogenannte Re-Stenose, auswirken.

Weiterhin kann ein erfindungsgemäßes Körperimplantat besser verformbar und insbesondere in einem Gefäß expandierbar sein.

### Bezugszeichenliste

- 1: Körperimplantat
- 10, 700: Textilstruktur (Geflecht)
- 12, 712: erstes Fasermaterial
- 14, 714: zweites Fasermaterial
- 20: Flechtmaschine
- 22: erste Klöppel
- 24: zweite Klöppel
- 716: abgewandelte Textilstruktur
- 800: Formkombination
- 802: erste Textilstruktur
- 804: zweite Textilstruktur 804
- 806: dritte Textilstruktur
- 900: Hauptstruktur
- 902: Folgestruktur 902

## Patentansprüche

1. Verfahren zum Herstellen eines Körperimplantats (1) mit den Schritten:
Erzeugen einer Textilstruktur (10) aus zumindest einem ersten Fasermaterial (12);
Durchführen einer ersten Formgebung an der Textilstruktur (10);
Entfernen eines Teils der Fasern des ersten Fasermaterials (12), und
Ersetzen durch ein zweites Fasermaterial (14) und/oder Wiedereinsetzen des ersten Fasermaterials (12) nach Durchführung eines weiteren Bearbeitungsschritts an dem entfernten Teil der Fasern des ersten Fasermaterials (12).

2. Verfahren zum Herstellen eines Körperimplantats mit folgenden Schritten:
Erzeugen einer Textilstruktur (700) oder eines Geflechts aus einem ersten Fasermaterial (712);
Durchführen einer ersten Formgebung an der Textilstruktur (700) oder dem Geflecht beispielsweise durch eine Wärmebehandlung;
Öffnen der Textilstruktur (700) aus dem ersten Fasermaterial (712);
Bereitstellen mindestens eines zweiten Fasermaterials (714); und
Herstellen einer abgewandelten Textilstruktur (716) aus dem ersten Fasermaterial (712) kombiniert mit dem mindestens zweiten Fasermaterial (714).

3. Verfahren nach Anspruch 1 oder 2, des Weiteren mit dem Schritt des Durchführens einer zweiten Formgebung an dem zweiten Fasermaterial (14, 714), wobei die zweite Formgebung so konfiguriert ist, dass nur das zweite Fasermaterial (14, 714) eine Formgebung erhält.

4. Verfahren nach Anspruch 3, wobei die zweite Formgebung bei einer niedrigeren Temperatur als die erste Formgebung durchgeführt wird.

5. Verfahren nach Anspruch 3 oder 4, wobei die zweite Formgebung an der aus den zwei Fasermaterialien (12, 712; 14, 714) erzeugten Textilstruktur (10, 716) durchgeführt wird oder vor dem Einsetzen des zweiten Fasermaterials (14, 714) separat durchgeführt wird.

6. Verfahren nach einem der vorherigen Ansprüche, wobei die Textilstruktur (10, 700, 716) durch Textilverarbeitung, Flechten, Weben, Wirken oder Stricken erzeugt wird.

7. Verfahren nach einem der vorherigen Ansprüche, wobei die Textilstruktur (10, 716) zumindest ein drittes Fasermaterial aufweist.

8. Verfahren nach einem der vorherigen Ansprüche, wobei zumindest eines der Fasermaterialien (12, 712; 14, 714) vor dem endgültigen Einbinden in die Textilstruktur (10, 716) elektropoliert oder beschichtet wird.

9. Verfahren nach einem der vorherigen Ansprüche, wobei das erste Fasermaterial (12, 712) Nitinol aufweist und das zweite (14, 714) und/oder dritte Fasermaterial eines aus einem Polymer, einem bioabbaubaren Polymer und Wolfram aufweist.

10. Verfahren nach einem der vorherigen Ansprüche, wobei zumindest eines der Fasermaterialien (12, 712; 14, 714) Formgedächtniseigenschaften aufweist.

11. Verfahren nach einem der vorherigen Ansprüche, wobei das erste Fasermaterial (12, 712) ein wärmebehandelbares Metall oder ein wärmebehandelbares Nichtmetall ist.

12. Verfahren nach einem der vorherigen Ansprüche, wobei das zweite Fasermaterial (14, 714) und das dritte Fasermaterial aus Metall und/oder Nichtmetall sind und/oder aus beschichtetem Garn sind.

13. Verfahren nach einem der vorherigen Ansprüche, wobei
- das erste Fasermaterial (12, 712) teilweise mit dem zweiten Fasermaterial (14, 714) und/oder dem dritten Fasermaterial verbunden ist und
- das zweiten Fasermaterial (14, 714) und/oder das dritte Fasermaterial nach Verlassen einer Hauptstruktur (900) eine eigene Folgestruktur (902) bilden.

14. Verfahren nach einem der oben genannten Ansprüche, wobei das zweite Fasermaterial (14, 714) denselben Werkstoff wie das erste Fasermaterial (12, 712) aufweist, und wobei das zweite Fasermaterial (14, 714) einer von der dem ersten Fasermaterial (12, 712) verschiedenen Formgebung unterzogen wurde und/oder verschiedene Eigenschaften aufweist und/oder unterschiedlich bearbeitet wurde.

15. Verfahren nach einem der oben genannten Ansprüche, wobei das zweite Fasermaterial (14, 714) einen von dem ersten Fasermaterial (12, 712) verschiedenen, zweiten Werkstoff aufweist, und wobei das zweite Fasermaterial (14, 714) von der dem ersten Fasermaterial (12, 712) verschiedenen Formgebung unterzogen wird.

16. Verfahren nach einem der vorherigen Ansprüche, wobei das zweite Fasermaterial (14, 714) und/oder das dritte Fasermaterial ein Verbindungselement zwischen zumindest zwei Textilstrukturen (802, 804, 806) bilden.

## Claims

1. Method for the production of a body implant (1), comprising the steps of:
creating a textile structure (10) from at least one first fiber material (12);
carrying out a first shaping on the textile structure (10);
removing a part of the fibers of the first fiber material (12), and
replacing by a second fiber material (14) and/or reinserting the first fiber material (12) after carrying out another processing step on the removed part of the fibers of the first fiber material (12).

2. Method for the production of a body implant, comprising the following steps:
creating a textile structure (700) or a meshwork from a first fiber material (712);
carrying out a first shaping on the textile structure (700) or the meshwork, for instance by means of a heat treatment;
opening the textile structure (700) made of the first fiber material (712);
providing at least a second fiber material (714); and
creating a modified textile structure (716) from the first fiber material (712) combined with the at least second fiber material (714).

3. Method according to claim 1 or 2, moreover comprising the step of carrying out a second shaping on the second fiber material (14, 714), the second shaping being configured in such a way that only the second fiber material (14, 714) is subject to shaping.

4. Method according to claim 3, wherein the second shaping is carried out at a lower temperature than the first shaping.

5. Method according to claim 3 or 4, wherein the second shaping is carried out on the textile structure (10, 716) created from the two fiber materials (12, 712; 14, 714) or is being carried out separately before inserting the second fiber material (14,714).

6. Method according to one of the previous claims, wherein the textile structure (10, 700, 716) is created by textile processing, braiding, weaving, warping or knitting.

7. Method according to one of the previous claims, wherein the textile structure (10, 716) has at least a third fiber material.

8. Method according to one of the previous claims, wherein at least one of the fiber materials (12, 712; 14, 714) is electropolished or coated before the final integration into the textile structure (10, 716).

9. Method according to one of the previous claims, wherein the first fiber material (12, 712) contains nitinol and the second fiber material (14, 714) and/or third fiber material contains one of a polymer, a biodegradable polymer and tungsten.

10. Method according to one of the previous claims, wherein at least one of the fiber materials (12, 712; 14, 714) has shape memory properties.

11. Method according to one of the previous claims, wherein the first fiber material (12, 712) is a heat-treatable metal or a heat-treatable nonmetal.

12. Method according to one of the previous claims, wherein the second fiber material (14, 714) and the third fiber material are made of metal and/or nonmetal and/or coated yarn.

13. Method according to one of the previous claims, wherein
- the first fiber material (12, 712) is partly combined with the second fiber material (14, 714) and/or the third fiber material and
- the second fiber material (14, 714) and/or the third fiber material form an own subsequent structure (902) after leaving a main structure (900).

14. Method according to one of the previous claims, wherein the second fiber material (14, 714) has the same material as the first fiber material (12, 712), and wherein the second fiber material (14, 714) has been subject to a shaping different from the one of the first material (12, 712) and/or has different properties and/or has been treated differently.

15. Method according to one of the previous claims, wherein the second fiber material (14, 714) has a second material different from the first fiber material (12, 712), and wherein the second fiber material (14, 714) is subject to the treatment different from the one of the first fiber material (12, 712).

16. Method according to one of the previous claims, wherein the second fiber material (14, 714) and/or the third fiber material form a connecting element between at least two textile structures (802, 804, 806).

## Revendications

1. Procédé de fabrication d'un implant corporel (1) avec les étapes :
génération d'une structure en textile (10) à partir d'au moins un premier matériau fibreux (12) ;
réalisation d'un premier façonnage sur la structure en textile (10) ;
élimination d'une partie des fibres du premier matériau fibreux (12), et
remplacement par un deuxième matériau fibreux (14) et/ou réutilisation du premier matériau fibreux (12) après réalisation d'une autre étape de traitement sur la partie éliminée des fibres du premier matériau fibreux (12).

2. Procédé de fabrication d'un implant corporel avec les étapes suivantes :
génération d'une structure en textile (700) ou d'un tissage à partir d'un premier matériau fibreux (712) ;
réalisation d'un premier façonnage sur la structure en textile (700) ou le tissage par exemple par un traitement thermique ;
ouverture de la structure en textile (700) constituée du premier matériau fibreux (712) ;
mise à disposition d'au moins un deuxième matériau fibreux (714) ; et
fabrication d'une structure en textile modifiée (716) à partir du premier matériau fibreux (712) combiné avec l'au moins un deuxième matériau fibreux (714).

3. Procédé selon la revendication 1 ou 2, en outre avec l'étape de la réalisation d'un second façonnage sur le deuxième matériau fibreux (14, 714), dans lequel le second façonnage est configuré de sorte que seul le deuxième matériau fibreux (14, 714) obtient un façonnage.

4. Procédé selon la revendication 3, dans lequel le second façonnage est réalisé à une température plus basse que le premier façonnage.

5. Procédé selon la revendication 3 ou 4, dans lequel le second façonnage est réalisé sur la structure en textile (10, 716) générée à partir des deux matériaux fibreux (12, 712 ; 14, 714) ou est réalisé séparément avant l'utilisation du deuxième matériau fibreux (14, 714).

6. Procédé selon une des revendications précédentes, dans lequel la structure en textile (10, 700, 716) est générée par transformation de textile, tressage, tissage, tricot ou tricotage.

7. Procédé selon une des revendications précédentes, dans lequel la structure en textile (10, 716) présente au moins un troisième matériau fibreux.

8. Procédé selon une des revendications précédentes, dans lequel au moins un des matériaux fibreux (12, 712 ; 14, 714) est électropoli ou enduit avant le liage définitif dans la structure en textile (10, 716).

9. Procédé selon une des revendications précédentes, dans lequel le premier matériau fibreux (12, 712) présente du nitinol et le deuxième (14, 714) et/ou troisième matériau fibreux présente un matériau parmi un polymère, un polymère biodégradable et du tungstène.

10. Procédé selon une des revendications précédentes, dans lequel au moins un des matériaux fibreux (12, 712 ; 14, 714) présente des propriétés de mémoire de forme.

11. Procédé selon une des revendications précédentes, dans lequel le premier matériau fibreux (12, 712) est un métal pouvant subir un traitement thermique ou un non-métal pouvant subir un traitement thermique.

12. Procédé selon une des revendications précédentes, dans lequel le deuxième matériau fibreux (14, 714) et le troisième matériau fibreux sont en métal et/ou non-métal et/ou sont en fil enduit.

13. Procédé selon une des revendications précédentes, dans lequel
- le premier matériau fibreux (12, 712) est partiellement connecté au deuxième matériau fibreux (14, 714) et/ou au troisième matériau fibreux et
- le deuxième matériau fibreux (14, 714) et/ou le troisième matériau fibreux forment une structure consécutive propre (902) après avoir quitté une structure principale (900).

14. Procédé selon une des revendications susmentionnées, dans lequel le deuxième matériau fibreux (14, 714) présente le même matériau que le premier matériau fibreux (12, 712), et dans lequel le deuxième matériau fibreux (14, 714) a été soumis à un façonnage différent de celui au premier matériau fibreux (12, 712) et/ou présente différentes propriétés et/ou a été traité différemment.

15. Procédé selon une des revendications susmentionnées, dans lequel le deuxième matériau fibreux (14, 714) présente un deuxième matériau différent du premier matériau fibreux (12, 712), et dans lequel le deuxième matériau fibreux (14, 714) a été soumis à un façonnage différent de celui au premier matériau fibreux (12,712).

16. Procédé selon une des revendications précédentes, dans lequel le deuxième matériau fibreux (14, 714) et/ou le troisième matériau fibreux forment un élément de connexion entre au moins deux structures en textile (802, 804, 806).
